# EUROPEAN PATENT SPECIFICATION

(11) **EP 1 595 537 B1**
(45) Date of publication and mention of the grant of the patent: **08.08.2007**
(21) Application number: 04712586.9
(22) Date of filing: 19.02.2004
(51) Int. Cl.: A61K 31/155, A61K 31/085, A61K 33/30, A61P 31/04

(54) **BUCCAL CAVITY TREATMENT COMPOSITION AND CORRESPONDING USES THEREOF**
ZUSAMMENSETZUNG ZUR BEHANDLUNG DER MUNDHÖHLE UND DEREN ENTSPRECHENDE VERWENDUNGEN
COMPOSITION POUR LE TRAITEMENT DE LA CAVITE BUCCALE ET UTILISATIONS CORRESPONDANTES

(30) Priority: 21.02.2003 ES 200300423
(43) Date of publication of application: 16.11.2005
(73) Proprietor: Laboratorios Kin, S.A., 08018 Barcelona (ES)
(72) Inventor: BALASCH RISUENO, Joaquin, E-08018 Barcelona (ES); SANCHO RIERA, Enriqueta, E-08029 Barcelona (ES); ARGUDO CARRERAS, Eva, E-08980 Sant Feliu de Llobregat (Barcelo (ES)
(74) Representative: Curell Aguilà, Marcelino
(86) International application number: PCT/ES2004/000075
(87) International publication number: WO 2004/073702

(56) References cited:
- EP-A2- 0 161 898
- WO-A1-00/51559
- WO-A1-94/12150
- WO-A1-94/26258
- WO-A1-97/25085
- ES-A- 2 137 139
- US-A- 5 015 467

## Description

### Field of the invention

This invention relates to a composition for treatment of the oral cavity.

### State of the art

Compositions are known for treatment of the oral cavity, containing derivatives of chlorhexidine. Chlorhexidine has anti-microbial and antiseptic characteristics, which are useful for treatment of the oral cavity. However, chlorhexidine tends to provoke discoloration of the teeth, this logically being an undesirable side effect.

Compositions are known for treatment of the oral cavity in which the concentration of chlorhexidine has been reduced (usually 0.12% in weight of chlorhexidine digluconate) and another component has been added to compensate the reduction in concentration of chlorhexidine. For example, the document ES P9801260 describes the combination of chlorhexidine digluconate with a zinc salt. However, it is still necessary to reach a compromise between the anti-microbial efficiency of a given composition and its tendency to discolour the teeth.

### Summary of the invention

The objective of the present invention is to propose a new composition which improves anti-microbial efficiency and has minimal tendency to discolour the teeth. This objective is achieved by means of a composition for treatment of the oral cavity such as indicated at the beginning of this specification and characterised in that it comprises the combination of a chlorhexidine derivative with triclosan and with a zinc salt.

In fact, it has been observed that the combination of chlorhexidine with triclosan and a zinc salt generates a synergetic effect with respect to the anti-microbial effectiveness of these components. It is consequently possible to achieve compositions with a given anti-microbial effect and which have a much lesser concentration of chlorhexidine than that of a composition designed to have the same anti-microbial effect and which contains only chlorhexidine. As a result it is possible to prepare compounds with highly effective anti-microbial properties and with contents in chlorhexidine lower than those known in the state of the art. This has a clear repercussion on the tendency to discolour the teeth, said tendency being in consequence reduced.

Preferably the chlorhexidine derivative is chlorhexidine digluconate, and advantageously the chlorhexidine digluconate is present in a concentration of between 0.01 and 0.2% in weight with respect to the total weight of the composition. It is particularly advantageous that the concentration of chlorhexidine digluconate be between 0.01 and 0.1% in weight with respect to the total weight of the composition and in particular that the concentration be between 0.04 and 0.06% in weight with respect to the total weight of the composition. Specifically, it has been observed that with 0.05% in weight with respect to the total weight of the composition, and with contents in triclosan and zinc salt as specified below, an anti-microbial efficiency can be obtained which is equivalent to a composition which contains only chlorhexidine digluconate in a concentration of 0.12% in weight with respect to the total weight of the composition.

As concerns triclosan it is present preferably in a concentration of between 0.05 and 1% in weight with respect to the total weight of the composition. In particular concentrations of triclosan of between 0.1 and 0.4% are advantageous.

Advantageously the zinc salt is zinc lactate (for example in solutions) or zinc citrate (for example in pastes), and the composition preferably has a Zn ion concentration of between 0.01 and 4% in weight with respect to the total weight of the composition. In particular Zn ion concentrations of between 0.05 and 0.2% are advantageous.

Compositions in accordance with the invention are preferably used in the preparation of toothpaste, gels and/or oral mouthwashes, and preferably for anti-plaque and gingivitis treatments.

### Detailed description of some embodiments of the invention

The following describes an example of a comparative study between a composition in accordance with the invention (which specifically comprises 0.05% chlorhexidine digluconate, 0.2% triclosan, 0.38% zinc lactate) and a composition known in the state of the art and commonplace in the market, which will be referred to below as the positive standard, and which contains chlorhexidine digluconate at 0.12%. The study extends to two other alternative compositions. The 4 compositions studied are thus:

**Table 1: Compositions studied**

| Product | Composition |
|---|---|
| P1 | CHX 0.05%, T 0.2%, LZ 0.38% |
| P2 | CHX 0.12% |
| P3 | T 0.2%, LZ 0.38% |
| P4 | CHX 0.05%, T 0.2% |

| | |
|---|---|
| CHX = chlorhexidine digluconate T = triclosan LZ = zinc lactate | |

Percentages are in weight with respect to the total weight of the composition.

### Material and methods:

This was a clinically controlled triple-blind crossed-over test. Test subjects were 10 adult volunteers who did not have oral or systemic diseases. Each of the test subjects consecutively used the four test products during 4 consecutive days, without brushing, with a window period of 3 days between each product during which the test subjects resumed their habitual oral hygiene practice. During each period of 4 days testing each volunteer mouthwashed twice per day, morning and night, with 15cm³ of gargle solution, for 1 minute, without using any other oral hygiene product, and without brushing their teeth. During the window period of 3 days the volunteers brushed their teeth according to their normal habits with toothpaste containing no anti-plaque product.

At the beginning of each test period of 4 days any remains of calculus in the upper and lower front teeth (13 to 23, and 33 to 43) was first removed, plaque was revealed, and completely eliminated with bicarbonate spray.

At the end of each test period of 4 days plaque was revealed, and two photographs were taken in a standard manner, which were then marked with the subject code and product code.

### Evaluation of the results:

The photographs taken of each subject at the end of 4 days use of each gargling solution, used without brushing, were developed and enlarged, and plaque was measured according to the following point system:
0. Absence of plaque
1. Discontinuous points of plaque in the cervical margin of the tooth
2. Continuous ribbon of plaque less than 1 mm wide at the cervical margin of the tooth
3. Ribbon of plaque wider than 1 mm, but which covers less than 1/3 of the crown
4. Plaque covering a minimum of 1/3 but less than 2/3 of the crown
5. Plaque covering 2/3 or more of the crown.

In addition a mean index was calculated for all teeth for all subjects (named the Turesky index) reached by adding all points obtained with each product and dividing them by the total number of teeth tested (120 teeth).

All the photos were examined and marked by the same person, and 5% of the dental surfaces were duplicate marked, at the end of the evaluation, in order to verify intraexaminer consistency in evaluation, which was found to be 85%, demonstrating very high consistency.

The data obtained was analysed statistically, taking the code for each subject and the plaque index with each of the products. So as to avoid loss of discrimination, the Turesky index was not calculated per person, but rather the sum of indices of each tooth was given as total points for plaque for each person with each product.

In analysing the data obtained it could be noted that the points obtained by one of the volunteers (subject 1) differed considerably from the trends of the other nine volunteers, since said subject displayed the highest and lowest plaque indexes with products contrary to the rest of the subjects. For this reason it was decided that the data should be analysed for the 10 volunteers, and for 9 volunteers (subjects 2 to 10, dismissing subject 1).

Descriptive statistics were compiled, finding the Turesky index averages for each product. For analysis of the differences between groups an analysis of the variance for repeated measures was carried out, to see if there were significant differences between the 4 products. In addition the significance of the differences between pairs of products (1-2, 1-3, 1-4, 2-3, 2-4 and 3-4) was analysed by means of the t-test for paired data.

Results obtained:

**Table 2: Total plaque results per product and subject (S1-S10: Subject 1 - Subject 10)**

| | S1 | S2 | S3 | S4 | S5 | S6 | S7 | S8 | S9 | S10 |
|---|---|---|---|---|---|---|---|---|---|---|
| P1 | 31 | 11 | 20 | 34 | 5 | 16 | 26 | 18 | 24 | 43 |
| P2 | 23 | 17 | 23 | 35 | 10 | 13 | 28 | 19 | 18 | 34 |
| P3 | 26 | 27 | 21 | 33 | 10 | 23 | 38 | 20 | 24 | 41 |
| P4 | 19 | 22 | 24 | 36 | 22 | 16 | 29 | 28 | 32 | 46 |

Figures in bold show the maximum value for each individual and figures underlined show the minimum value for each individual.

**Table 3: Mean plaque index for each product, considering the 10 subjects**

| Product | Composition | Turesky index |
|---|---|---|
| P1 | CHX 0.05%, T 0.2%, LZ 0.38% | 1.90 |
| P2 | CHX 0.12% | 1.83 |
| P3 | T 0.2%, LZ 0.38% | 2.19 |
| P4 | CHX 0.05%, T 0.2% | 2.28 |

**Table 4: Mean plaque index for each product, considering 9 subjects**

| Product | Composition | Turesky index |
|---|---|---|
| P1 | CHX 0.05%, T 0.2%, LZ 0.38% | 1.82 |
| P2 | CHX 0.12% | 1.82 |
| P3 | T 0.2%, LZ 0.38% | 2.19 |
| P4 | CHX 0.05%, T 0.2% | 2.36 |

From Table 2, in which results for all subjects and products are summarised, it can be appreciated that, despite the large differences between such, all subjects have the highest plaque index with products 3 (3 subjects) or 4 (6 subjects), with the exception of subject N° 1. Additionally, the majority of subjects have minimum indexes with products 1 (5 subjects) or 2 (3 subjects) with the exception of subject N° 4, and again subject N° 1. It is for this reason that the decision was made to analyse the results including and excluding subject N° 1.

Tables 3 and 4 show the Turesky index for each product, said index being appreciably lower for products 1 and 2 than for products 3 and 4.

It can be seen that product 1 (0.05% chlorhexidine digluconate, 0.2% triclosan, 0.38% zinc lactate) does not show any significant differences with respect to product 2 (0.12% chlorhexidine digluconate) in the test as to plaque without brushing, and is significantly more efficient than products 3 (0.2% triclosan, 0.38% zinc lactate) and 4 (0.05% chlorhexidine digluconate, 0.2% triclosan). As concerns chlorhexidine digluconate at 0.12% it is more efficient than products 3 (0.2% triclosan, 0.38% zinc lactate) and 4 (0.05% chlorhexidine digluconate, 0.2% triclosan).

Which is to say, the same efficiency in plaque control has been obtained with the composition in accordance with the invention (with 0.05% chlorhexidine digluconate) as with a composition having 0.12% chlorhexidine digluconate.

Additionally it should be taken into account that compositions with 0.12% chlorhexidine are commonplace in the market, practically defining a commercial standard having known anti-bacterial and anti-plaque characteristics suitable for the treatment and prevention of gingivitis and bacterial plaque in a wide range of patients or users. It is therefore particularly significant to be able to offer an equivalent composition, which has equivalent anti-microbial characteristics (and therefore is also appropriate for treatment and prevention of gingivitis and bacterial plaque in a wide spectrum of patients). The invention also achieves this, since presenting combinations of chlorhexidine digluconate with triclosan and a zinc salt that are specifically equivalent to a composition with 0.12% chlorhexidine digluconate. An example of such equivalent compositions are those that comprise between 0.04% and 0.06% chlorhexidine digluconate, between 0.1 and 0.4% triclosan and between 0.05% and 0.2% Zn ion.

### Examples of formulations

| Formulation for a toothpaste: | |
|---|---|
| Triclosan | 0'30 % |
| Chlorhexidine digluconate | 0'05 % |
| Zinc citrate | 0'50 % |
| Sodium fluoride | 0'22 % |
| Xylitol | 1'00 % |
| Cocamidopropyl betaine | 2'00 % |
| Xantan gum | 1'20 % |
| Propylenglicol | 1'00 % |
| Sodium saccharine | 0'07 % |
| Sodium methylparaben | 0'15 % |
| Glycerol | 15'00 % |
| Sorbitol (sol. 70 %) | 27'00 % |
| Precipitated silica | 14'00 % |
| Titanium dioxide | 1'20 % |
| Flavouring | 0'70 % |
| Purified water as required for | 100 g |

| Formulation for a mouthwash: | |
|---|---|
| Triclosan | 0'20 % |
| Chlorhexidine digluconate | 0'05 % |
| Zinc lactate | 0'38 % |
| Xylitol | 1'00% |
| Sodium saccharine | 0'02 % |
| Sorbitol (sol. 70 %) | 10'00 % |
| Glycerol | 5'00 % |
| Propylenglicol | 2'50% |
| Hydroxylated and polyethoxylated castor oil PEG-40 | 1'60 % |
| Flavouring | 0'15 % |
| Purified water as required for | 100 ml |

Preferably the mouthwash comprises EDTA, for example disodium EDTA. EDTA is a sequester agent and the addition thereof allows the solution to be stabilised, avoiding the formation of precipitates. Advantageously the composition comprises between 0.05 and 0.2% in weight of disodium EDTA with respect to the total weight of the composition, and more advantageously still the concentration of disodium EDTA is 0.1%. The mouthwash preferably has a pH approximately equal to 6. Preferably this pH is obtained by adding the necessary quantity of sodium hydroxide.

A preferable formulation for a mouthwash with EDTA is the following:

| | |
|---|---|
| Triclosan | 0'20 % |
| Chlorhexidine digluconate | 0'05 % |
| Zinc lactate | 0'38 % |
| Xylitol | 1'00 % |
| Sodium saccharine | 0'02 % |
| Sorbitol (sol. 70 %) | 10'00 % |
| Glycerol | 5'00 % |
| Propylenglicol | 2'50 % |
| Hydroxylated and polyethoxylated castor oil PEG-40 | 1'60 % |
| Flavouring | 0'15 % |
| Disodium EDTA | 0'10 % |
| Sodium hydroxide as required for | pH 6 |
| Purified water as required for | 100 ml |

| Formulation for a gel: | |
|---|---|
| Triclosan | 0'30 % |
| Chlorhexidine digluconate | 0'05 % |
| Zinc citrate | 0'50 % |
| Sodium fluoride | 0'22 % |
| Xylitol | 1'00 % |
| Hidroxyethylcelulose | 1'00 % |
| Propylenglicol | 1'00 % |
| Sodium saccharine | 0'07 % |
| Sodium methylparaben | 0'15 % |
| Glycerol | 15'00 % |
| Sorbitol (sol. 70 %) | 27'00 % |
| Precipitated silica | 14'0 % |
| Flavouring | 0'70 % |
| Purified water as required for | 100 g |

The gel will include cocamidopropylbetaine if used for brushing the teeth, and will not include such if used as gel for topical application in oral treatment.

## Claims

1. Composition for treatment of the oral cavity, **characterised in that** it comprises the combination of a derivative of chlorhexidine with triclosan and with a zinc salt.

2. Composition according to claim 1, **characterised in that** said derivative of chlorhexidine is chlorhexidine digluconate, and **in that** the chlorhexidine digluconate is present in a concentration of between 0.01 and 0.2% in weight with respect to the total weight of the composition.

3. Composition according to one of claims 1 or 2, **characterised in that** said triclosan is present in a concentration of between 0.05 and 1% in weight with respect to the total weight of the composition.

4. Composition according to any of claims 1 to 3, **characterised in that** said zinc salt is zinc lactate or zinc citrate.

5. Composition according to any of claims 1 to 4, **characterised in that** it has a Zn ion concentration of between 0.01 and 4% in weight with respect to the total weight of the composition.

6. Composition according to any of claims 2 to 5, **characterised in that** said chlorhexidine digluconate is present in a concentration of between 0.01 and 0.1 % in weight with respect to the total weight of the composition.

7. Composition according to claim 6, **characterised in that** said chlorhexidine digluconate is present in a concentration of between 0.04 and 0.06% in weight with respect to the total weight of the composition.

8. Composition according to any of claims 3 to 7, **characterised in that** said triclosan is present in a concentration of between 0.1 and 0.4% in weight with respect to the total weight of the composition.

9. Composition according to any of claims 5 to 8, **characterised in that** it has a Zn ion concentration of between 0.05 and 0.2% in weight with respect to the total weight of the composition.

10. Composition according to any of claims 1 to 9, **characterised in that** it is a mouthwash and **in that** it comprises disodium EDTA.

11. Composition according to claim 10, **characterised in that** it has a concentration of EDTA of between 0.05 and 0.2% in weight with respect to the total weight of the composition.

12. Composition according to claim 11, **characterised in that** it has a concentration of EDTA of 0.1 % in weight with respect to the total weight of the composition, and **in that** it has a pH equal to 6.

13. Use of a composition according to any of claims 1 to 9 for the preparation of a toothpaste.

14. Use of a composition according to any of claims 1 to 9 for preparation of a gel.

15. Use of a composition according to any of claims 1 to 9 for the preparation of a mouthwash.

16. Use of a composition according to any of claims 1 to 12 for the preparation of a medicine for a treatment of the group formed by anti-plaque treatment and gingivitis treatment.

## Patentansprüche

1. Zusammensetzung zur Behandlung der Mundhöhle, **dadurch gekennzeichnet, dass** sie die Kombination eines Derivats von Chlorhexidin mit Triclosan und einem Zinksalz umfasst.

2. Zusammensetzung gemäß Anspruch 1, **dadurch gekennzeichnet, dass** das Derivat von Chlorhexidin Chlorhexidindigluconat ist und das Chlorhexidindigluconat in einer Konzentration von 0,01 bis 0,2 Gew.-%, bezogen auf das Gesamtgewicht der Zusammensetzung vorliegt.

3. Zusammensetzung gemäß einem der Ansprüche 1 oder 2, **dadurch gekennzeichnet, dass** das Triclosan in einer Konzentration von 0,05 bis 1 Gew.-%, bezogen auf das Gesamtgewicht der Zusammensetzung vorliegt.

4. Zusammensetzung gemäß einem der Ansprüche 1 bis 3, **dadurch gekennzeichnet, dass** das Zinksalz Zinklaktat oder Zinkcitrat ist.

5. Zusammensetzung gemäß einem der Ansprüche 1 bis 4, **dadurch gekennzeichnet, dass** sie eine Konzentration an Zinkionen von 0,01 bis 4 Gew.-%, bezogen auf das Gesamtgewicht der Zusammensetzung aufweist.

6. Zusammensetzung gemäß einem der Ansprüche 2 bis 5, **dadurch gekennzeichnet, dass** das Chlorhexidindigluconat in einer Konzentration von 0,01 bis 0,1 Gew.-%, bezogen auf das Gesamtgewicht der Zusammensetzung vorliegt.

7. Zusammensetzung gemäß Anspruch 6, **dadurch gekennzeichnet, dass** das Chlorhexidindigluconat in einer Konzentration von 0,04 bis 0,06 Gew.-%, bezogen auf das Gesamtgewicht der Zusammensetzung vorliegt.

8. Zusammensetzung gemäß einem der Ansprüche 3 bis 7, **dadurch gekennzeichnet, dass** das Triclosan in einer Konzentration von 0,1 bis 0,4 Gew.-%, bezogen auf das Gesamtgewicht der Zusammensetzung vorliegt.

9. Zusammensetzung gemäß einem der Ansprüche 5 bis 8, **dadurch gekennzeichnet, dass** sie eine Konzentration an Zinkionen von 0,05 bis 0,2 Gew.-%, bezogen auf das Gesamtgewicht der Zusammensetzung aufweist.

10. Zusammensetzung gemäß einem der Ansprüche 1 bis 9, **dadurch gekennzeichnet, dass** sie ein Mundwasser ist und sie Dinatrium-EDTA umfasst.

11. Zusammensetzung gemäß Anspruch 10, **dadurch gekennzeichnet, dass** sie eine Konzentration an EDTA von 0,05 bis 0,2 Gew.-%, bezogen auf das Gesamtgewicht der Zusammensetzung aufweist.

12. Zusammensetzung gemäß Anspruch 11, **dadurch gekennzeichnet, dass** sie eine Konzentration an EDTA von 0,1 Gew.-%, bezogen auf das Gesamtgewicht der Zusammensetzung, und einen pH-Wert von 6 aufweist.

13. Verwendung einer Zusammensetzung gemäß einem der Ansprüche 1 bis 9 für die Herstellung einer Zahnpasta.

14. Verwendung einer Zusammensetzung gemäß einem der Ansprüche 1 bis 9 für die Herstellung eines Gels.

15. Verwendung einer Zusammensetzung gemäß einem der Ansprüche 1 bis 9 für die Herstellung eines Mundwassers.

16. Verwendung einer Zusammensetzung gemäß einem der Ansprüche 1 bis 12 für die Herstellung eines Arzneimittels für eine Behandlung ausgewählt aus der Gruppe, bestehend aus Antiplaquebehandlung und Behandlung von Zahnfleischentzündung.

## Revendications

1. Composition pour le traitement de la cavité ovale, **caractérisée en ce qu'**elle comprend la combinaison d'un dérivé de chlorhexidine avec du triclosan et avec un sel de zinc.

2. Composition selon la revendication 1, **caractérisée en ce que** ledit dérivé de chlorhexidine est le digluconate de chlorhexidine, et **en ce que** le digluconate de chlorhexidine est présent en une concentration entre 0,01 et 0,2 % en poids par rapport au poids total de la composition.

3. Composition selon l'une des revendications 1 ou 2, **caractérisée en ce que** ledit triclosan est présent en une concentration entre 0,05 et 1 % en poids par rapport au poids total de la composition.

4. Composition selon l'une quelconque des revendications 1 à 3, **caractérisée en ce que** ledit sel de zinc est le lactate de zinc ou le citrate de zinc.

5. Composition selon l'une quelconque des revendications 1 à 4, **caractérisée en ce qu'**elle a une concentration en ion Zn entre 0,01 et 4 % en poids par rapport au poids total de la composition.

6. Composition selon l'une quelconque des revendications 2 à 5, **caractérisée en ce que** ledit digluconate de chlorhexidine est présent en une concentration entre 0,01 et 0,1 % en poids par rapport au poids total de la composition.

7. Composition selon la revendication 6, **caractérisée en ce que** ledit digluconate de chlorhexidine est présent en une concentration entre 0,04 et 0,06 % en poids par rapport au poids total de la composition.

8. Composition selon l'une quelconque des revendications 3 à 7, **caractérisée en ce que** ledit triclosan est présent en une concentration entre 0,1 et 0,4 % en poids par rapport au poids total de la composition.

9. Composition selon l'une quelconque des revendications 5 à 8, **caractérisée en ce qu'**elle a une concentration en ion Zn entre 0,05 et 0,2 % en poids total par rapport au poids total de la composition.

10. Composition selon l'une quelconque des revendications 1 à 9, **caractérisée en ce qu'**elle est un rince-bouche et **en ce qu'**elle comprend de l'EDTA disodique.

11. Composition selon la revendication 10, **caractérisée en ce qu'**elle a une concentration en EDTA entre 0,05 et 0,2 % en poids par rapport au poids total de la composition.

12. Composition selon la revendication 11, **caractérisée en ce qu'**elle a une concentration en EDTA de 0,1 % en poids par rapport au poids total de la composition et **en ce qu'**elle a un pH égal à 6.

13. Utilisation d'une composition sur l'une quelconque des revendications 1 à 9 pour la préparation d'une pâte dentifrice.

14. Utilisation d'une composition selon l'une quelconque des revendications 1 à 9 pour la préparation d'un gel.

15. Utilisation d'une composition selon l'une quelconque des revendications 1 à 9 pour la préparation d'un rince-bouche.

16. Utilisation d'une composition selon l'une quelconque des revendications 1 à 12 pour la préparation d'un médicament pour le traitement du groupe formé par le traitement antiplaque et le traitement de gingivite.
